# EUROPEAN PATENT APPLICATION

(11) **EP 4 631 933 A1**
(43) Date of publication of application: **15.10.2025**
(21) Application number: 23901057.2
(22) Date of filing: 05.12.2023
(51) Int. Cl.: C07D 239/95, A61K 31/517, A61P 35/00, A23L 33/10

(54) **NOVEL 2-((TRANS-4-(4-ARYLOXY)CYCLOHEXYL)AMINO)QUINAZOLINONE DERIVATIVE AND METHOD FOR PREPARING SAME**

(30) Priority: 07.12.2022 KR 20220169785
(71) Applicant: NexThera Co., Ltd., Busan 48931 (KR)
(72) Inventor: YUN, Hwayoung, Busan 46241 (KR); JEONG, Jee-Yeong, Busan 488118 (KR); KWAK, Jinsook, Busan 46241 (KR); KIM, Min Jung, Busan 46241 (KR)
(74) Representative: Mewburn Ellis LLP
(86) International application number: PCT/KR2023/019875
(87) International publication number: WO 2024/123038

(57) **Abstract**

The objective of the present invention is to provide a novel 2-((trans-4-(4-aryloxy)cyclohexyl)amino)quinazolinone derivative which exhibits anti-cancer activity through eIF2α phosphorylation efficacy. The novel synthesized derivative exhibits an eIF2α phosphorylation effect and cancer cell proliferation inhibitory activity in vitro, and thus can be used as a metabolic anti-cancer drug in pharmaceutical and health functional food compositions for preventing and ameliorating leukemia and other rare cancers such as breast cancer, brain tumor, and sarcoma.

## Description

### Technical Field

The present disclosure provides a novel 2-((trans-4-(4-aryloxy)cyclohexyl)amino)quinazolinone derivative and a preparation method thereof.

### Background Art

Research on the cause of cancer cell death by regulating the synthesis of proteins related to the metabolism of cancer cells has recently been in the spotlight. Tumor-associated proteins are encoded by so-called "weak" mRNAs, but while its expression is negligible in normal cells, the expression increases sharply in cancer cells.

As a target to regulate these oncogenic factors, phosphorylation of an alpha subunit of eukaryotic initiation factor 2 (eIF2α), a representative factor of eukaryotic translation initiation, can be proposed. eIF2α is phosphorylated by four kinases HRI, PERK, GCN2, and PKR, and each kinase is activated by cellular stress. As a result, phosphorylated eIF2α may suppress protein biosynthesis.

During cellular carcinogenesis, eIF2 is overexpressed and rapidly accelerates translation of weak mRNAs. Since phosphorylation of eIF2α can inhibit translation of weak mRNA, it may also result in inhibition of expression of oncogenic factors. However, to date, no small molecule candidate showing eIF2α phosphorylation efficacy has been reported to have entered the clinical trial. Therefore, research on novel substances with superior eIF2α phosphorylation efficacy is needed, and development of metabolic anticancer agents using the mechanism is necessary.

### Disclosure of the Invention

### Technical Goals

An object of the present disclosure is to provide a compound selected from a 2-((trans-4-(4-aryloxy)cyclohexyl)amino)quinazolinone derivative compound, a pharmaceutically acceptable salt thereof, a solvate thereof, or a stereoisomer thereof.

Another object of the present disclosure is to provide a pharmaceutical composition for preventing or treating a cancer, including the compound as an active ingredient.

Another object of the present disclosure is to provide a pharmaceutical composition for a metabolic anticancer agent, including the compound as an active ingredient.

Another object of the present disclosure is to provide a method of inhibiting cancer cell metabolism, including administering the compound.

Another object of the present disclosure is to provide a health functional food composition for preventing or ameliorating a cancer, including the compound as an active ingredient.

### Technical Solutions

To achieve the above object, the present disclosure provides a compound selected from a 2-((trans-4-(4-aryloxy)cyclohexyl)amino)quinazolinone derivative compound represented by the following Chemical Formula 1, a pharmaceutically acceptable salt thereof, a solvate thereof, or a stereoisomer thereof.

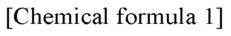

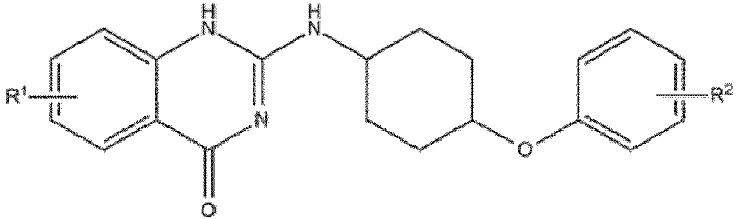

In the Chemical Formula 1,
R¹ and R² may be the same or different and each selected from the group consisting of hydrogen (H), halogen, trifluoromethyl (CF₃), nitro (NO₂), (C1~C4)alkoxy, trifluoromethoxy (OCF₃), cyano (CN), nitro (NO₂), (C1~C4)alkylsulfonyl, and aminosulfonyl (SO₂NH₂).

In addition, the present disclosure provides a pharmaceutical composition for preventing or treating a cancer, including the compound as an active ingredient.

In addition, the present disclosure provides a pharmaceutical composition for a metabolic anticancer agent, including the compound as an active ingredient.

In addition, the present disclosure provides a method of inhibiting cancer cell metabolism, including administering the compound.

In addition, the present disclosure provides a health functional food composition for preventing or ameliorating a cancer, including the compound as an active ingredient.

### Advantageous Effects

The present disclosure relates to a compound selected from a 2-((trans-4-(4-aryloxy)cyclohexyl)amino)quinazolinone derivative compound, a pharmaceutically acceptable salt thereof, a solvate thereof, or a stereoisomer thereof, which exhibits an anticancer activity through eIF2α phosphorylation efficacy, wherein a novel synthesized derivative exhibits an eIF2α phosphorylation effect and an in-vitro cancer cell proliferation inhibitory activity and thus may be utilized as a pharmaceutical and health functional food composition for preventing and ameliorating leukemia, as well as rare cancers such as breast cancer, brain tumors, and sarcoma, as a metabolic anticancer agent.

### Brief Description of Drawings

FIG. 1 shows a design strategy for a novel 2-((trans-4-(4-aryloxy)cyclohexyl)amino)quinazolinone derivative.
FIG. 2 shows identification of an effect of a novel 2-((trans-4-(4-aryloxy)cyclohexyl)amino)quinazolinone derivative on eIF2α phosphorylation.

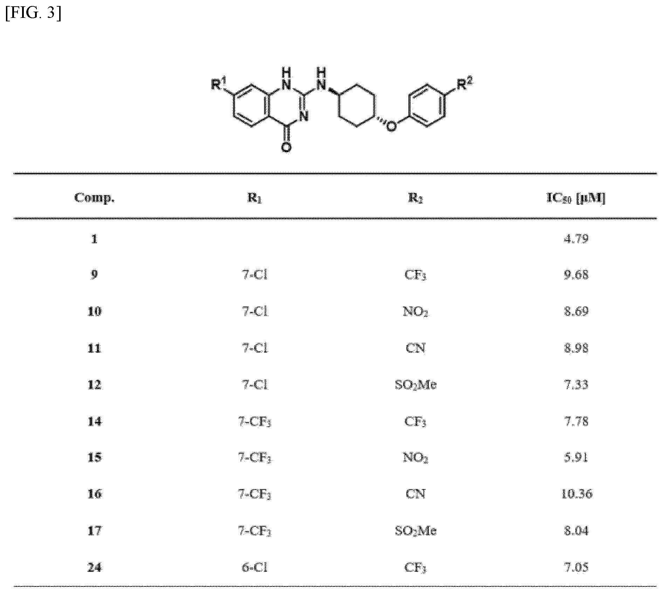

FIG. 3 shows a result of identifying an antiproliferative activity of a novel 2-((trans-4-(4-aryloxy)cyclohexyl)amino)quinazolinone derivative.

### Best Mode for Carrying Out the Invention

Hereinafter, the present disclosure will be described in more detail.

A key step in initiation of protein synthesis is formation of a complex consisting of eIF2, GTP, and Met-tRNAi. Since the complex plays an important role in performing normal cellular functions and is known to cause various diseases when abnormal regulation takes place, it has been drawing attention as a target for development of new therapeutic agents, and thus inventors of the present disclosure completed the present disclosure by synthesizing a 2-((trans-4-(4-aryloxy)cyclohexyl)amino)quinazolinone derivative compound having various substituents to identify that the novel derivative thus synthesized exhibits an eIF2α phosphorylation effect and an in-vitro cancer cell proliferation inhibitory activity.

To achieve the above object, the present disclosure provides a compound selected from a 2-((trans-4-(4-aryloxy)cyclohexyl)amino)quinazolinone derivative compound represented by the following Chemical Formula 1, a pharmaceutically acceptable salt thereof, a solvate thereof, or a stereoisomer thereof.

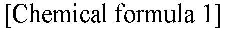

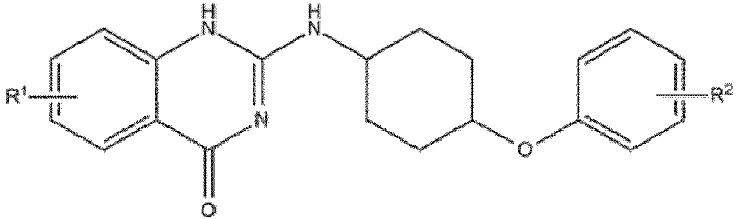

In the Chemical Formula 1, R¹ and R² may be the same or different and each selected from the group consisting of hydrogen (H), halogen, trifluoromethyl (CF₃), nitro (NO₂), (C1~C4)alkoxy, trifluoromethoxy (OCF₃), cyano (CN), nitro (NO₂), (C1~C4)alkylsulfonyl, and aminosulfonyl (SO₂NH₂).

Preferably, the 2-((trans-4-(4-aryloxy)cyclohexyl)amino)quinazolinone derivative compound in Chemical Formula 1 may have R¹ selected from the group consisting of hydrogen (H), halogen, trifluoromethyl (CF₃), nitro (NO₂), and (C1~C2)alkoxy, and R² selected from the group consisting of chlorine (Cl), trifluoromethyl (CF₃), trifluoromethoxy (OCF₃), cyano (CN), nitro (NO₂), (C1~C2)alkylsulfonyl, and aminosulfonyl (SO₂NH₂).

Preferably, the 2-((trans-4-(4-aryloxy)cyclohexyl)amino)quinazolinone derivative compound in the Chemical Formula 1 may have R¹ selected from the group consisting of hydrogen (H), fluorine (F), chlorine (Cl), trifluoromethyl (CF₃), and nitro (NO₂), and R² selected from the group consisting of chlorine (Cl), trifluoromethyl (CF₃), trifluoromethoxy (OCF₃), cyano (CN), nitro (NO₂), methylsulfonyl (SO₂Me), andaminosulfonyl (SO₂NH₂).

Preferably, the 2-((trans-4-(4-aryloxy)cyclohexyl)amino)quinazolinone derivative compound may be represented by the following Chemical Formula 1-1.

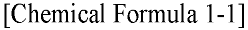

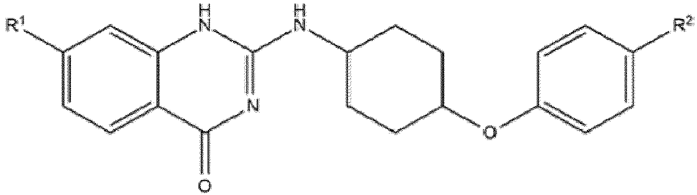

In the Chemical Formula 1-1, R¹ may be chlorine (Cl) or trifluoromethyl (CF₃), and R² may be selected from the group consisting of trifluoromethyl (CF₃), nitro (NO₂), cyano (CN), and methylsulfonyl (SO₂Me).

In an example embodiment, the 2-((trans-4-(4-aryloxy)cyclohexyl)amino)quinazolinone derivative compound may be selected from the group consisting of, but is not limited to, 2-(((1,4-trans)-4-(4-(trifluoromethyl)phenoxy)cyclohexyl)amino)quinazolin-4(1H)-one; 2-(((1,4-trans)-4-(4-nitrophenoxy)cyclohexyl)amino)quinazolin-4(1H)-one; 4-(((1,4-trans)-4-((4-oxo-1,4-dihydroquinazolin-2-yl)amino)cyclohexyl)oxy)benzonitrile; 2-(((1,4-trans)-4-(4-(methylsulfonyl)phenoxy)cyclohexyl)amino)quinazolin-4(1H)-one; 7-fluoro-2-(((1,4-trans)-4-(4-(trifluoromethyl)phenoxy)cyclohexyl)amino)quinazolin-4(1H)-one; 7-fluoro-2-(((1,4-trans)-4-(4-nitrophenoxy)cyclohexyl)amino)quinazolin-4(1H)-one; 4-(((1,4-trans)-4-((7-fluoro-4-oxo-1,4-dihydroquinazolin-2-yl)amino)cyclohexyl)oxy)benzonitrile; 7-fluoro-2-(((1,4-trans)-4-(4-(methylsulfonyl)phenoxy)cyclohexyl)amino)quinazolin-4(1H)-one; 7-chloro-2-(((1,4-trans)-4-(4-(trifluoromethyl)phenoxy)cyclohexyl)amino)quinazolin-4(1H)-one; 7-chloro-2-(((1,4-trans)-4-(4-nitrophenoxy)cyclohexyl)amino)quinazolin-4(1H)-one; 4-(((1,4-trans)-4-((7-chloro-4-oxo-1,4-dihydroquinazolin-2-yl)amino)cyclohexyl)oxy)benzonitrile; 7-chloro-2-(((1,4-trans)-4-(4-(methylsulfonyl)phenoxy)cyclohexyl)amino)quinazolin-4(1H)-one; 4-(((1,4-trans)-4-((7-chloro-4-oxo-1,4-dihydroquinazolin-2-yl)amino)cyclohexyl)oxy)benzenesulfonamide; 7-(trifluoromethyl)-2-(((1,4-trans)-4-(4-(trifluoromethyl)phenoxy)cyclohexyl)amino)quinazolin-4(1H)-one; 2-(((1,4-trans)-4-(4-nitrophenoxy)cyclohexyl)amino)-7-(trifluoromethyl)quinazolin-4(1H)-one; 4-(((1,4-trans)-4-((4-oxo-7-(trifluoromethyl)-1,4-dihydroquinazolin-2-yl)amino)cyclohexyl)oxy)benzonitrile; 2-(((1,4-trans)-4-(4-(methylsulfonyl)phenoxy)cyclohexyl)amino)-7-(trifluoromethyl)quinazolin-4(1H)-one; 4-(((1,4-trans)-4-((4-oxo-7-(trifluoromethyl)-1,4-dihydroquinazolin-2-yl)amino)cyclohexyl)oxy)benzenesulfonamide; 2-(((1,4-trans)-4-(4-chlorophenoxy)cyclohexyl)amino)-7-(trifluoromethyl)quinazolin-4(1H)-one; 2-(((1,4-trans)-4-(4-chlorophenoxy)cyclohexyl)amino)-7-(trifluoromethyl)quinazolin-4(1H)-one; 7-nitro-2-(((1,4-trans)-4-(4-(trifluoromethyl)phenoxy)cyclohexyl)amino)quinazolin-4(1H)-one; 7-nitro-2-(((1,4-trans)-4-(4-nitrophenoxy)cyclohexyl)amino)quinazolin-4(1H)-one; 4-(((1,4-trans)-4-((7-nitro-4-oxo-1,4-dihydroquinazolin-2-yl)amino)cyclohexyl)oxy)benzonitrile; 2-(((1,4-trans)-4-(4-(methylsulfonyl)phenoxy)cyclohexyl)amino)-7-nitroquinazolin-4(1H)-one; 6-chloro-2-(((1,4-trans)-4-(4-(trifluoromethyl)phenoxy)cyclohexyl)amino)quinazolin-4(1H)-one; 6-chloro-2-(((1,4-trans)-4-(4-nitrophenoxy)cyclohexyl)amino)quinazolin-4(1H)-one; 4-(((1,4-trans)-4-((6-chloro-4-oxo-1,4-dihydroquinazolin-2-yl)amino)cyclohexyl)oxy)benzonitrile; and 6-chloro-2-(((1,4-trans)-4-(4-(methylsulfonyl)phenoxy)cyclohexyl)amino)quinazolin-4(1H)-one.

In addition, the present disclosure provides a pharmaceutical composition for preventing or treating a cancer, including the compound as an active ingredient.

Preferably, the compound according to the present disclosure may promote phosphorylation of eukaryotic translation initiation factor 2α (eIF2α).

Preferably, the cancer may be, but is not limited to, blood cancer, breast cancer, sarcoma, or brain cancer.

In addition, the present disclosure provides a pharmaceutical composition for a metabolic anticancer agent, including the compound as an active ingredient.

In addition, the present disclosure provides a method of inhibiting cancer cell metabolism, including administering the compound.

The compound may kill cancer cells by blocking their metabolism through promotion of phosphorylation of eukaryotic translation initiation factor 2α (eIF2α).

The metabolic anticancer agent is called the 4^{th} generation anticancer agent following chemotherapies, targeted anticancer agents, and immunotherapies, and is an anticancer agent that selectively kills only cancer cells by blocking the metabolic process of cancer cells, unlike existing anticancer treatments, in a way of blocking the cancer cell metabolic activity that causes more active cell division than normal cells, particularly it lowers the cancer cell metabolism by more than 50% with little damage to normal cells in the process of inhibiting cancer cell growth.

As used herein, the pharmaceutically acceptable salt may be one or more basic salts selected from the group consisting of sodium salt, potassium salt, calcium salt, lithium salt, magnesium salt, cesium salt, aminium salt, ammonium salt, triethylaminium salt, and pyridinium salt, but is not limited thereto.

In addition, it is stated that the pharmaceutically acceptable salt may be one or more acidic salts selected from the group consisting of hydrochloric acid, hydrobromic acid, sulfuric acid, sulfurous acid, phosphoric acid, citric acid, acetic acid, maleic acid, fumaric acid, gluconic acid, methanesulfonic acid, benzenesulfonic acid, camphorsulfonic acid, oxalic acid, malonic acid, glutaric acid, acetic acid, glycolic acid, succinic acid, tartaric acid, 4-toluenesulfonic acid, galacturonic acid, embonic acid, glutamic acid, citric acid, and aspartic acid, but is not limited thereto.

The pharmaceutical composition of the present disclosure may include, in addition to the components described above, pharmaceutically acceptable carriers, excipients, or diluents for administration. The carriers, excipients, and diluents may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

The pharmaceutical composition of the present disclosure may be used by being formulated in the form of oral formulations such as powders, granules, tablets, capsules, suspensions, emulsions, syrups, and aerosols, external preparations, suppositories, or sterile injectable solutions, respectively, according to conventional methods. Specifically, when formulated, it may be prepared using diluents or excipients such as fillers, extenders, binders, wetting agents, disintegrating agents, and surfactants that are commonly used. Solid preparations for oral administration include, but are not limited to, tablets, pills, powders, granules, and capsules. These solid preparations may be prepared by mixing, in addition to the active ingredient, at least one or more excipients, such as starch, calcium carbonate, sucrose, lactose, and gelatin. Additionally, in addition to simple excipients, lubricants such as magnesium stearate and talc may also be used. In addition to liquid and liquid paraffin for oral administration, it may be prepared by adding various excipients such as wetting agents, sweeteners, flavoring agents, and preservatives. Preparations for parenteral administration include sterile aqueous solutions, non-aqueous solvents, suspensions, emulsions, lyophilized preparations, and suppositories. Non-aqueous solvents and suspensions that may be used include propylene glycol, polyethylene glycol, vegetable oils such as olive oil, and injectable esters such as ethyl oleate. Base materials for suppositories that may be used include witepsol, macrogol, tween 61, cacao butter, laurin fat, and glycerogelatin.

It is stated that the pharmaceutical composition of the present disclosure may be prepared as an oral or parenteral preparation and may be administered by oral, intravenous, intracerebroventricular, intradermal, intramuscular, intraperitoneal, nasal, or epidural routes, but is not limited thereto.

The appropriate dosage of the pharmaceutical composition of the present disclosure varies depending on the patient's condition and weight, degree of diseases, drug form, and time, and may be appropriately selected by a person skilled in the art, such that the daily dosage of the composition is preferably 0.01 mg/kg to 100 mg/kg and may be administered once a day or several times a day as needed.

In addition, the present disclosure provides a health functional food composition for preventing or ameliorating a cancer, including the compound as an active ingredient.

The above health functional food composition may contain various nutrients, vitamins, minerals (electrolytes), flavoring agents such as synthetic flavoring agents and natural flavoring agents, coloring agents and thickening agents (cheese, chocolate, etc.), pectic acid and salts thereof, alginic acid and salts thereof, organic acids, protective colloid thickeners, pH regulators, stabilizers, preservatives, glycerin, alcohol, and carbonating agents used in carbonated beverages. In addition, it may also contain pulp for production of natural fruit juice, synthetic fruit juice, and vegetable drinks. These ingredients may be used independently or in combination. In addition, the health functional food composition may be in any one form of meat, sausage, bread, chocolate, candy, snacks, confectionery, pizza, ramen, gum, ice cream, soup, beverage, tea, functional water, drink, alcohol, and vitamin complex.

In addition, the health functional food composition may further include food additives, and the suitability as the food additive is determined by the standards and criteria associated with corresponding items according to the general rules and general test methods of Korean Food Additives Codex approved by the Ministry of Food and Drug Safety, unless otherwise stipulated.

The items listed in the Korean Food Additives Codex may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents.

Here, in the process of manufacturing the health functional food composition, the content of the composition according to the present disclosure added to food may be appropriately adjusted as needed.

### Modes for Carrying Out the Invention

Hereinafter, the present disclosure will be described in more detail through examples and experimental examples. These examples and experimental examples are only intended to explain the present disclosure more specifically, and it will be apparent to those skilled in the art that the scope of the present disclosure is not limited by these examples and experimental examples according to the gist of the present disclosure.

### [Synthesis Example] Synthesis of 2-((trans-4-(4-aryloxy)cyclohexyl)amino)quinazolinone

### (1) Urea cyclization

Phenol containing a mixture of aminobenzoic acid (1.0 eq) and urea (5.0 eq) was heated to 200°C. After stirring for 17 hours, the reaction mixture was cooled to 100°C, and then water was added dropwise. The reaction mixture was cooled to room temperature, and then the resulting solid was filtered and washed/dried with ethanol to obtain quinazolinedione.

### (2-1) Dichlorination using N,N-diethylaniline

N,N-diethylaniline (30 mol%) was added to a stirred POCl₃ (10.0 eq) solution containing the above quinazolinedione (1.0 eq) and heated to 180°C. After 5 to 17 hours, the reaction mixture was cooled to 0°C. The reaction mixture was poured into ice water and quenched with 6 N sodium hydroxide (NaOH). The organic layer was extracted three times with dichloromethane (CH₂Cl₂), dried with magnesium sulfate (MgSO₄) for concentration, and purified by flash column chromatography on silica gel to obtain dichloroquinazoline.

### (2-2) Dichlorination using PCl₅

A mixture of the quinazolinedione (1.0 eq) and PCl₅ (3.7 eq) in POCl₃ (10.0 eq) was heated to 180°C. After stirring for 5 to 17 hours, the reaction mixture was cooled to 0°C. The reaction mixture was poured into ice water and quenched with 6 N sodium hydroxide (NaOH). The organic layer was extracted three times with dichloromethane (CH₂Cl₂), dried with magnesium sulfate (MgSO₄) for concentration, and purified by flash column chromatography on silica gel to obtain dichloroquinazoline.

### (3) Mono-hydroxylation

A mixture of the above dichloroquinazoline (1.0 eq) in 1 N NaOH/THF (1:1) was stirred at room temperature. After 6 hours, the reaction mixture was adjusted to pH 5 with acetic acid, and the precipitated solid was filtered and dried to obtain mono-hydroxyquinazolinone.

### (4) Nucleophilic aromatic substitution

Diisopropylethylamine (3.0 eq) was added to a stirred DMF solution containing the mono-hydroxyquinazoline (1.0 eq) and 4-(aryloxy)cyclohexylamine (1.5 eq) in DMF, and the mixture was heated to 80°C. After 17 hours, the reaction mixture was cooled to room temperature and diluted with ethyl acetate (EtOAc). The organic layer was washed with water (3 times) and dried with magnesium sulfate (MgSO₄). The resulting residue was concentrated in vacuo and purified by flash column chromatography on silica gel to obtain 2-((trans-4-(4-aryloxy)cyclohexyl)amino)quinazolinone.

### [Synthesis Example 2] Specific synthesis example of a 2-((trans-4-(4-aryloxy)cyclohexyl)amino)quinazolinone derivative

### (1) 2-(((1,4-trans)-4-(4-(Trifluoromethyl)phenoxy)cyclohexyl)amino)quinazolin-4(1H)-one (hereinafter referred to as Compound 1)

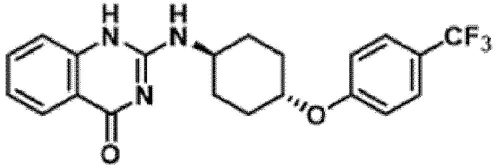

(1,4-trans)-4-(4-(Trifluoromethyl)phenoxy)cyclohexan-1-amine (152 mg, 0.56 mmol) and N,N-diisopropylethylamine (203 µL, 1.17 mmol) were added to a DMF solution (3 mL) containing 2-chloroquinazolin-4(1H)-one (69.6 mg, 0.39 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 21.0 mg (13%) of Compound 1 in white solid.

¹H NMR (DMSO-*d*₆, 400 MHz)*δ* 10.62 (s, 1H), 7.88 (d, 1H, *J =* 8.0 Hz), 7.62 (d, 2H, *J* = 8.3 Hz), 7.55 (m, 1H), 7.26 (d, 1H, *J =* 8.4 Hz), 7.14 (d, 2H, *J =* 8.2 Hz), 7.09 (t, 1H, *J =* 7.4 Hz), 6.25 (d, 1H, *J =* 7.0 Hz), 4.50 (m, 1H), 3.87 (m, 1H), 2.08 (m, 2H), 2.06 (m, 2H), 1.55 (m, 2H), 1.44 (m, 2H); ¹³C NMR (DMSO-*d*₆, 100 MHz)*δ* 161.9, 160.3, 151.1, 149.8, 134.2, 131.6, 128.6, 127.0, 127.0, 126.0, 125.9, 124.6, 123.3, 121.6, 121.3, 121.0, 120.7, 120.4, 117.4, 115.8, 74.4, 47.7, 29.5, 29.4.

### (2) 2-(((1,4-trans)-4-(4-Nitrophenoxy)cyclohexyl)amino)quinazolin-4(1H)-one (hereinafter referred to as Compound 2)

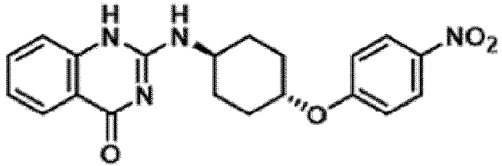

(1,4-trans)-4-(4-Nitrophenoxy)cyclohexan-1-amine (195 mg, 0.83 mmol) and N,N-diisopropylethylamine (287 µL, 1.65 mmol) were added to DMF solution (3 mL) containing 2-chloroquinazolin-4(1H)-one (100 mg, 0.55 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 43.8 mg (21%) of Compound 2 in brown solid.

¹H NMR (DMSO-*d*₆, 400 MHz)*δ* 10.61 (s, 1H), 8.18 (d, 2H, *J =* 9.3 Hz), 7.88 (d, 1H, *J =* 7.8 Hz), 7.55 (t, 1H, *J* = 7.7 Hz), 7.26 (d, 1H, *J =* 8.2 Hz), 7.17 (d, 2H, *J =* 9.2 Hz), 7.09 (t, 1H, *J =* 7.3 Hz), 6.26 (d, 1H, *J =* 6.3 Hz), 4.59 (m, 1H), 3.88 (m, 1H), 2.08 (m, 4H), 1.58 (m, 2H), 1.46 (m, 2H); ¹³C NMR (DMSO-*d*₆, 125 MHz)*δ* 163.0, 161.9, 151.2, 149.9, 140.5, 134.3, 126.0, 126.0, 124.7, 121.7, 117.4, 115.8, 75.2, 47.7, 40.02, 29.5, 29.4.

### (3) 4-(((1,4-trans)-4-((4-Oxo-1,4-dihydroquinazolin-2-yl)amino)cyclohexyl)oxy)benzonitrile (hereinafter referred to as Compound 3)

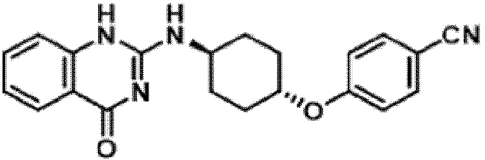

4-(((1,4-trans)-4-Aminocyclohexyl)oxy)benzonitrile (324 mg, 1.50 mmol) and N,N-diisopropylethylamine (523 µL, 3.00 mmol) were added to DMF solution (5 mL) containing 2-chloroquinazolin-4(1H)-one (180 mg, 1.00 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 100 mg (28%) of Compound 3 in ivory solid.

¹H NMR (DMSO-*d*₆, 500 MHz)*δ* 10.60 (s, 1H), 7.88 (d, 1H, *J =* 7.8 Hz), 7.74 (d, 2H, *J* = 8.7 Hz), 7.55 (t, 1H, *J =* 7.6 Hz), 7.26 (d, 1H, *J* = 8.2 Hz), 7.13 (d, 2H, *J* = 8.7 Hz), 7.09 (t, 1H, *J =* 7.4 Hz), 6.25 (d, 1H, *J =* 6.7 Hz), 4.52 (m, 1H), 3.87 (m, 1H), 2.07 (m, 2H), 2.06 (m, 2H), 1.55 (m, 2H), 1.44 (m, 2H); ¹³C NMR (DMSO-*d*₆, 125 MHz)*δ* 161.9, 161.1, 151.2, 149.9, 134.3, 126.0, 124.7, 121.7, 119.3, 117.4, 116.4, 102.5, 74.6, 47.7, 29.5, 29.4.

### (4) 2-(((1,4-trans)-4-(4-(Methylsulfonyl)phenoxy)cyclohexyl)amino)quinazolin-4(1H)-one (hereinafter referred to as Compound 4)

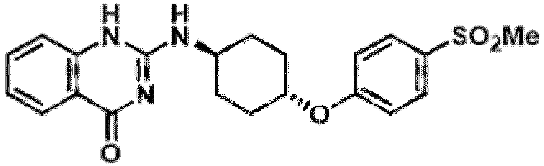

(1,4-trans)-4-(4-(Methylsulfonyl)phenoxy)cyclohexan-1-amine (222 mg, 0.83 mmol) and N,N-diisopropylethylamine (287 µL, 1.65 mmol) were added to DMF solution (5 mL) containing 2-chloroquinazolin-4(1H)-one (100 mg, 0.55 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 74.0 mg (32%) of Compound 4 in white solid.

¹H NMR (DMSO-*d*₆, 500 MHz)*δ* 10.60 (s, 1H), 7.88 (dd, 1H, *J =* 7.9, 1.6 Hz), 7.82 (d, 2H, *J =* 8.9 Hz), 7.55 (ddd, 1H, *J =* 8.2, 7.1, 1.7 Hz), 7.26 (d, 1H, *J =* 8.2 Hz), 7.19 (d, 2H, *J =* 9.0 Hz), 7.09 (t, 1H, *J =* 7.4 Hz), 6.25 (d, 1H, *J =* 6.9 Hz), 4.55 (m, 1H), 3.88 (m, 1H), 3.15 (s, 3H), 2.08 (m, 4H), 1.57 (m, 2H), 1.46 (m, 2H); ¹³C NMR (DMSO-*d*₆, 125 MHz)*δ* 161.9, 161.4, 151.2, 149.9, 134.3, 132.3, 129.3, 126.0, 124.7, 121.7, 117.4, 115.8, 74.6, 47.7, 44.0, 29.5, 29.4.

### (5) 7-Fluoro-2-(((1,4-trans)-4-(4-(trifluoromethyl)phenoxy)cyclohexyl)amino)quinazolin-4(1H)-one (hereinafter referred to as Compound 5)

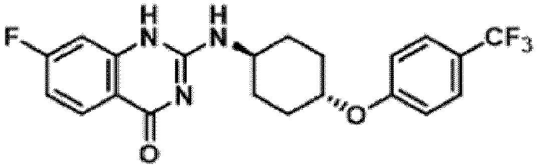

(1,4-trans)-4-(4-(Trifluoromethyl)phenoxy)cyclohexan-1-amine (194 mg, 0.75 mmol) and N,N-diisopropylethylamine (261 µL, 1.50 mmol) were added to DMF solution (5 mL) containing 2-chloro-7-fluoroquinazolin-4(1H)-one (100 mg, 0.50 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obatin 35.4 mg (17%) of Compound 5 in white solid.

¹H NMR (DMSO-*d*₆, 500 MHz)*δ* 10.61 (s, 1H), 7.91 (dd, 1H, *J =* 8.6, 6.8 Hz), 7.60 (d, 2H, *J* = 8.7 Hz), 7.13 (d, 2H, *J* = 8.6 Hz), 6.95 (dd, 1H, *J* = 11.0, 2.2 Hz), 6.89 (td, 1H, *J =* 8.6, 2.4 Hz), 6.35 (s, 1H), 4.49 (m, 1H), 3.88 (m, 1H), 2.07 (m, 4H), 1.55 (m, 2H), 1.45 (m, 2H); ¹³C NMR (DMSO-*d*₆, 125 MHz)*δ* 167.2, 165.2, 161.2, 160.4, 153.6, 153.4, 150.8, 129.0, 128.9, 127.9, 127.1, 127.0, 127.0, 127.0, 125.7, 123.6, 121.4, 121.3, 121.0, 120.8, 120.5, 115.9, 114.4, 110.1, 109.9, 109.5, 109.3, 74.4, 47.8, 29.4.

### (6) 7-Fluoro-2-(((1,4-trans)-4-(4-nitrophenoxy)cyclohexyl)amino)quinazolin-4(1H)-one (hereinafter referred to as Compound 6)

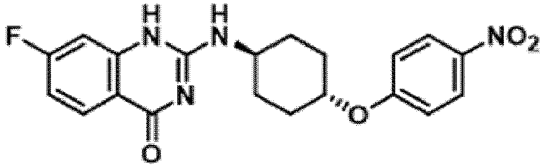

(1,4-trans)-4-(4-Nitrophenoxy)cyclohexan-1-amine (269 mg, 1.14 mmol) and N,N-diisopropylethylamine (397 µL, 2.28 mmol) were added to DMF solution (5 mL) containing 2-chloro-7-fluoroquinazolin-4(1H)-one (150 mg, 0.76 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 89.5 mg (30%) of Compound 6 in brown solid.

¹H NMR (DMSO-*d*₆, 500 MHz)*δ* 10.63 (s, 1H), 8.18 (d, 2H, *J =* 9.3 Hz), 7.92 (dd, 1H, *J =* 8.7, 6.8 Hz), 7.17 (d, 2H, *J =* 9.3 Hz), 6.97 (dd, 1H, *J =* 10.7, 1.5 Hz), 6.92 (td, 1H, *J =* 8.7, 2.2 Hz), 6.40 (s, 1H), 4.59 (m, 1H), 3.88 (m, 1H), 2.08 (m, 4H), 1.57 (m, 2H), 1.47 (m, 2H); ¹³C NMR (DMSO-*d*₆, 125 MHz)*δ* 167.2, 165.2, 162.9, 161.1, 153.5, 153.4, 150.7, 140.5, 128.9, 128.8, 126.0, 115.7, 114.4, 110.1, 109.9, 109.5, 109.3, 75.1, 47.7, 29.4, 29.3.

### *(7) 4-(((1,4-trans)-4-((7-Fluoro-4-oxo-1,4-dihydroquinazolin-2-yl)amino)cyclohexyl)oxy)benzonitrile (hereinafter referred to as Compound 7)

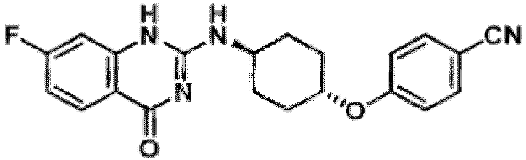

4-(((1,4-trans)-4-Aminocyclohexyl)oxy)benzonitrile (162 mg, 0.75 mmol) and N,N-diisopropylethylamine (261 µL, 1.50 mmol) were added to DMF solution (5 mL) containing 2-chloro-7-fluoroquinazolin-4(1H)-one (100 mg, 0.50 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 42.6 mg (22%) of Compound 7 in white solid.

¹H NMR (DMSO-*d*₆, 500 MHz)*δ* 7.92 (dd, 1H, *J* = 8.7, 6.7 Hz), 7.74 (d, 2H, *J* = 8.9 Hz), 7.13 (d, 2H, *J =* 8.9 Hz), 6.97 (dd, 1H, *J =* 11.0, 2.4 Hz), 6.92 (td, 1H, *J =* 8.7, 2.5 Hz), 6.49 (s, 1H), 4.52 (m, 1H), 3.87 (m, 1H), 2.06 (m, 4H), 1.54 (m, 2H), 1.45 (m, 2H); ¹³C NMR (DMSO-*d*₆, 125 MHz)*δ* 167.2, 165.3, 161.2, 161.1, 153.6, 153.5, 150.8, 134.3, 129.0, 128.9, 119.3, 116.4, 114.4, 110.2, 110.0, 109.5, 109.3, 102.6, 74.6, 47.8, 29.4.

### (8) 7-Fluoro-2-(((1,4-trans)-4-(4-(methylsulfonyl)phenoxy)cyclohexyl)amino)quinazolin-4(1H)-one (hereinafter referred to as Compound 8)

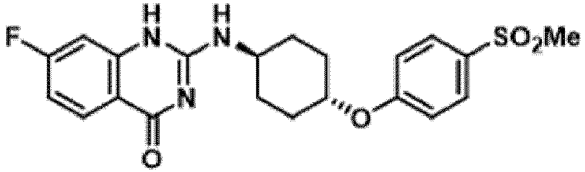

(1,4-trans)-4-(4-(Methylsulfonyl)phenoxy)cyclohexan-1-amine (202 mg, 0.75 mmol) and N,N-diisopropylethylamine (261 µL, 1.50 mmol) were added to DMF solution (5 mL) containing 2-chloro-7-fluoroquinazolin-4(1H)-one (100 mg, 0.50 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 37.6 mg (17%) of Compound 8 in white solid.

¹H NMR (DMSO-*d*₆, 500 MHz)*δ* 10.65 (s, 1H), 7.92 (d, 1H, *J =* 8.7, 6.8 Hz), 7.82 (d, 2H, *J =* 8.9 Hz), 7.18 (d, 2H, *J =* 8.9 Hz), 6.97 (dd, 1H, *J =* 10.9, 2.0 Hz), 6.92 (td, 1H, *J =* 8.6, 2.3 Hz), 6.45 (s, 1H), 4.54 (m, 1H), 3.88 (m, 1H), 3.15 (s, 3H), 2.07 (m, 4H), 1.56 (m, 2H), 1.46 (m, 2H); ¹³C NMR (DMSO-*d*₆, 125 MHz)*δ* 167.2, 165.2, 161.3, 161.2, 153.5, 153.4, 150.8, 132.3, 129.3, 128.9, 128.8, 115.8, 114.4, 110.1, 109.9, 109.4, 109.3, 74.6, 47.7, 44.0, 29.4.

### (9) 7-Chloro-2-(((1,4-trans)-4-(4-(trifluoromethyl)phenoxy)cyclohexyl)amino)quinazolin-4(1H)-one (hereinafter referred to as Compound 9)

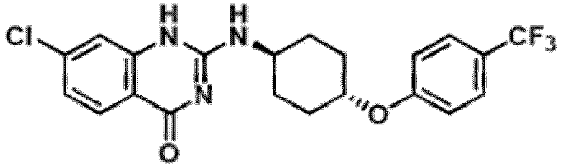

(1,4-trans)-4-(4-(Trifluoromethyl)phenoxy)cyclohexan-1-amine (362 mg, 1.40 mmol) and N,N-diisopropylethylamine (486 µL, 2.79 mmol) were added to DMF solution (7 mL) containing 2,7-dichloroquinazolin-4(1H)-one (200 mg, 0.93 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 68.0 mg (17%) of Compound 9 in white solid.

¹H NMR (DMSO-*d*₆, 400 MHz)*δ* 10.71 (s, 1H), 7.86 (m, 1H), 7.62 (d, 2H, *J =* 8.2 Hz), 7.26 (s, 1H), 7.14 (d, 2H, *J =* 8.2 Hz), 7.09 (d, 1H, *J =* 8.4 Hz), 6.42 (s, 1H), 4.50 (m, 1H), 3.87 (m, 1H), 2.06 (m, 4H), 1.49 (m, 4H); ¹³C NMR (DMSO-*d*₆, 125 MHz)*δ* 161.5, 160.5, 152.5, 150.9, 139.2, 128.2, 128.0, 127.2, 127.2, 127.2, 127.1, 125.9, 123.7, 122.0, 121.6, 121.4, 121.2, 120.9, 120.7, 116.2, 116.0, 74.5, 48.1, 29.6, 29.5.

### (10) 7-Chloro-2-(((1,4-trans)-4-(4-nitrophenoxy)cyclohexyl)amino)quinazolin-4(1H)-one (hereinafter referred to as Compound 10)

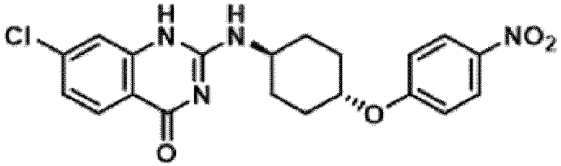

(1,4-trans)-4-(4-Nitrophenoxy)cyclohexan-1-amine (331 mg, 1.40 mmol) and N,N-diisopropylethylamine (486 µL, 2.79 mmol) were added to DMF solution (7 mL) containing 2,7-dichloroquinazolin-4(1H)-one (200 mg, 0.93 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 60.6 mg (16%) of Compound 10 in light brown solid.

¹H NMR (DMSO-*d*₆, 500 MHz)*δ* 10.71 (s, 1H), 8.19 (d, 2H, *J =* 9.3 Hz), 7.86 (d, 1H, *J =* 8.5 Hz), 7.26 (d, 1H, *J =* 1.5 Hz), 7.18 (d, 2H, *J =* 9.3 Hz), 7.10 (dd, 1H, *J =* 8.4, 1.8 Hz), 6.44 (s, 1H), 4.60 (m, 1H), 3.87 (m, 1H), 2.08 (m, 4H), 1.57 (m, 2H), 1.47 (m, 2H); ¹³C NMR (DMSO-*d*₆, 125 MHz)*δ* 162.9, 161.3, 152.4, 150.8, 140.5, 138.9, 128.0, 126.0, 123.6, 121.8, 116.2, 115.7, 75.1, 47.8, 37.4, 29.4.

*

### (11) 4-(((1,4-trans)-4-((7-Chloro-4-oxo-1,4-dihydroquinazolin-2-yl)amino)cyclohexyl)oxy)benzonitrile (hereinafter referred to as Compound 11)

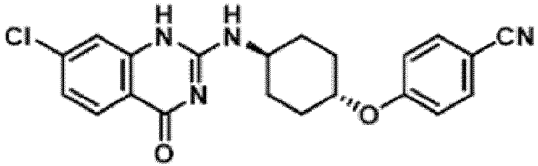

4-(((1,4-trans)-4-Aminocyclohexyl)oxy)benzonitrile (273 mg, 1.26 mmol) and N,N-diisopropylethylamine (439 µL, 2.52 mmol) were added to DMF solution (6 mL) containing 2,7-dichloroquinazolin-4(1H)-one (180 mg, 0.84 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 161 mg (49%) of Compound 11 in white solid.

¹H NMR (DMSO-*d*₆, 500 MHz)*δ* 10.36 (s, 1H), 7.85 (d, 1H, *J =* 8.5 Hz), 7.74 (d, 2H, *J* = 8.7 Hz), 7.26 (d, 1H, *J =* 1.2 Hz), 7.13 (d, 2H, *J =* 8.7 Hz), 7.09 (dd, 1H, *J =* 8.4, 1.4 Hz), 6.55 (m, 1H), 4.52 (m, 1H), 3.86 (m, 1H), 2.06 (m, 4H), 1.54 (m, 2H), 1.45 (m, 2H); ¹³C NMR (DMSO-*d*₆, 125 MHz)*δ* 161.5, 161.1, 152.4, 151.0, 138.9, 134.3, 128.1, 123.5, 121.8, 119.3, 116.4, 116.2, 102.5, 74.6, 47.9, 29.4.

### (12) 7-Chloro-2-(((1,4-trans)-4-(4-(methylsulfonyl)phenoxy)cyclohexyl)amino)quinazolin-4(1H)-one (hereinafter referred to as Compound 12)

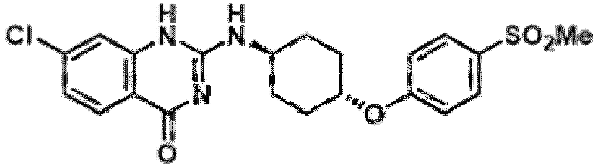

(1,4-trans)-4-(4-(Methylsulfonyl)phenoxy)cyclohexan-1-amine (190 mg, 0.71 mmol) and N,N-diisopropylethylamine (246 µL, 1.41 mmol) were added to DMF solution (3 mL) containing 2,7-dichloroquinazolin-4(1H)-one (100 mg, 0.47 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 114 mg (55%) of Compound 12 in ivory solid.

¹H NMR (DMSO-*d*₆, 500 MHz)*δ* 10.69 (s, 1H), 7.86 (d, 1H, *J =* 8.5 Hz), 7.82 (d, 2H, *J* = 8.7 Hz), 7.26 (s, 1H), 7.18 (d, 2H, *J* = 8.7 Hz), 7.09 (d, 1H, *J =* 8.5 Hz), 6.54 (s, 1H), 4.54 (m, 1H), 3.87 (m, 1H), 3.15 (s, 3H), 2.07 (m, 4H), 1.55 (m, 2H), 1.46 (m, 2H); ¹³C NMR (DMSO-*d*₆, 100 MHz)*δ* 161.6, 161.3, 152.4, 151.0, 138.8, 132.3, 129.3, 128.0, 123.5, 121.7, 116.2, 115.8, 74.6, 47.9, 44.0, 29.4.

### (13) 4-(((1,4-trans)-4-((7-Chloro-4-oxo-1,4-dihydroquinazolin-2-yl)amino)cyclohexyl)oxy)benzenesulfonamide (hereinafter referred to as Compound 13)

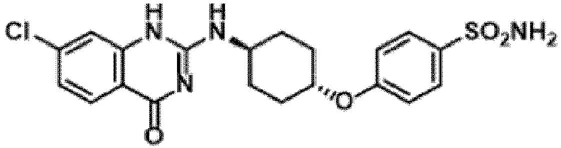

4-(((1,4-trans)-4-Aminocyclohexyl)oxy)benzenesulfonamide (150 mg, 0.56 mmol) and N,N-diisopropylethylamine (193 µL, 1.11 mmol) were added to DMF solution (2.5 mL) containing 2,7-dichloroquinazolin-4(1H)-one (79.5 mg, 0.37 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 43.9 mg (26%) of Compound 13 in white solid.

¹H NMR (DMSO-*d*₆, 500 MHz)*δ* 10.70 (s, 1H), 7.86 (d, 1H, *J =* 8.4 Hz), 7.73 (d, 2H, *J =* 8.5 Hz), 7.26 (s, 1H), 7.19 (s, 2H), 7.11 (d, 2H, *J =* 8.4 Hz), 7.09 (m, 1H), 6.43 (s, 1H), 4.49 (m, 1H), 3.86 (m, 1H), 2.06 (m, 4H), 1.54 (m, 2H), 1.45 (m, 2H); ¹³C NMR (DMSO-*d*₆, 125 MHz)*δ* 161.3, 159.9, 152.4, 150.8, 139.0, 136.0, 128.0, 127.8, 123.6, 121.7, 116.2, 115.4, 74.3, 47.9, 29.4.

### (14) 7-(Trifluoromethyl)-2-(((1,4-trans)-4-(4-(trifluoromethyl)phenoxy)cyclohexyl)amino)quinazolin-4(1H)-one (hereinafter referred to as Compound 14)

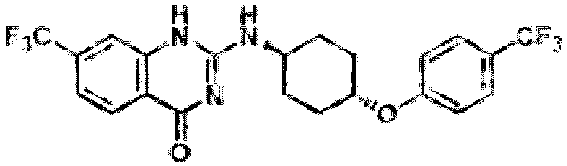

(1,4-trans)-4-(4-(Trifluoromethyl)phenoxy)cyclohexan-1-amine (190 mg, 0.71 mmol) and N,N-diisopropylethylamine (209 µL, 1.20 mmol) were added to DMF solution (3 mL) containing 2-chloro-7-(trifluoromethyl)quinazolin-4(1H)-one (100 mg, 0.40 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 112 mg (30%) of Compound 14 in white solid.

¹H NMR (DMSO-*d*₆, 500 MHz)*δ* 10.86 (s, 1H), 8.05 (d, 1H, *J =* 8.0 Hz), 7.62 (d, 2H, *J* = 8.2 Hz), 7.51 (s, 1H), 7.34 (d, 1H, *J =* 7.3 Hz), 7.14 (d, 2H, *J* = 8.2 Hz), 6.49 (s, 1H), 4.50 (m, 1H), 3.90 (m, 1H), 2.08 (m, 4H), 1.55 (m, 2H), 1.47 (m, 2H); ¹³C NMR (DMSO-*d*₆, 125 MHz)*δ* 161.2, 160.3, 151.4, 150.9, 134.5, 134.2, 134.0, 133.7, 127.9, 127.7, 127.1, 127.1, 127.0, 127.0, 127.0, 125.7, 124.9, 123.6, 122.8, 121.5, 121.4, 121.3, 121.0, 120.8, 120.6, 120.5, 120.1, 117.1, 115.9, 74.4, 48.0, 29.5, 29.4.

### (15) 2-(((1,4-trans)-4-(4-Nitrophenoxy)cyclohexyl)amino)-7-(trifluoromethyl)quinazolin-4(1H)-one (hereinafter referred to as Compound 15)

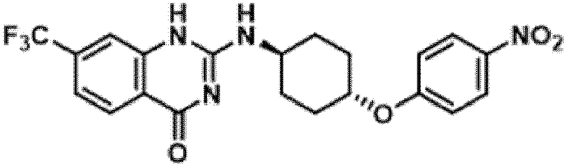

(1,4-trans)-4-(4-Nitrophenoxy)cyclohexan-1-amine (113 mg, 0.48 mmol) and N,N-diisopropylethylamine (167 µL, 0.96 mmol) were added to DMF solution (3 mL) containing 2-chloro-7-(trifluoromethyl)quinazolin-4(1H)-one (80.0 mg, 0.32 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 43.9 mg (30%) of Compound 15 in light yellow solid.

¹H NMR (DMSO-*d*₆, 500 MHz)*δ* 10.87 (s, 1H), 8.18 (d, 2H, *J* = 9.3 Hz), 8.05 (d, 1H, *J =* 8.3 Hz), 7.50 (s, 1H), 7.34 (d, 1H, *J =* 8.1 Hz), 7.17 (d, 2H, *J =* 9.4 Hz), 6.52 (s, 1H), 4.59 (m, 1H), 3.90 (m, 1H), 2.09 (m, 4H), 1.58 (m, 2H), 1.49 (m, 2H); ¹³C NMR (DMSO-*d*₆, 100 MHz)*δ* 162.9, 161.2, 151.3, 150.9, 140.5, 134.5, 134.2, 133.9, 133.6, 127.9, 127.6, 126.0, 125.2, 122.5, 121.4, 120.1, 119.7, 117.0, 115.7, 75.1, 47.9, 29.4, 29.3.

### (16) 4-(((1,4-trans)-4-((4-Oxo-7-(trifluoromethyl)-1,4-dihydroquinazolin-2-yl)amino)cyclohexyl)oxy)benzonitrile (hereinafter referred to as Compound 16)

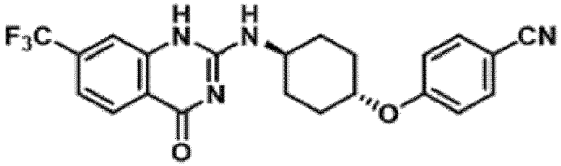

4-(((1,4-trans)-4-Aminocyclohexyl)oxy)benzonitrile (130 mg, 0.60 mmol) and N,N-diisopropylethylamine (209 µL, 1.20 mmol) were added to DMF solution (3 mL) containing 2-chloro-7-(trifluoromethyl)quinazolin-4(1H)-one (100 mg, 0.40 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 48.6 mg (28%) of Compound 16 in white solid.

¹H NMR (DMSO-*d*₆, 500 MHz)*δ* 11.11 (s, 1H), 8.05 (d, 1H, *J =* 8.2 Hz), 7.74 (d, 2H, *J =* 8.8 Hz), 7.49 (s, 1H), 7.33 (d, 1H, *J =* 8.2 Hz), 7.14 (d, 2H, *J =* 8.8 Hz), 6.92 (d, 1H, *J =* 4.9 Hz), 4.54 (m, 1H), 3.90 (m, 1H), 2.07 (m, 4H), 1.55 (m, 2H), 1.47 (m, 2H); ¹³C NMR (DMSO-*d*₆, 125 MHz)*δ* 161.0, 151.3, 151.0, 134.4, 134.2, 134.1, 133.9, 133.6, 127.6, 127.1, 124.9, 122.7, 121.4, 121.4, 120.6, 120.1, 119.2, 116.9, 116.3, 102.5, 74.4, 47.8, 29.2.

### (17) 2-(((1,4-trans)-4-(4-(Methylsulfonyl)phenoxy)cyclohexyl)amino)-7-(trifluoromethyl)quinazolin-4(1H)-one (hereinafter referred to as Compound 17)

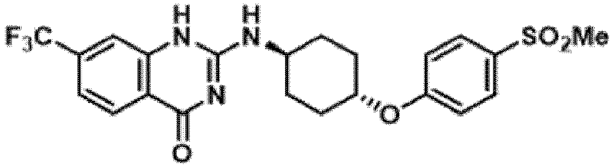

(1,4-trans)-4-(4-(Methylsulfonyl)phenoxy)cyclohexan-1-amine (129 mg, 0.48 mmol) and N,N-diisopropylethylamine (167 µL, 0.96 mmol) were added to DMF solution (3 mL) containing 2-chloro-7-(trifluoromethyl)quinazolin-4(1H)-one (80.0 mg, 0.32 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 37.4 mg (24%) of Compound 17 in ivory solid.

¹H NMR (DMSO-*d*₆, 500 MHz)*δ* 8.05 (d, 1H, *J =* 8.2 Hz), 7.82 (d, 2H, *J =* 8.8 Hz), 7.50 (s, 1H), 7.33 (d, 1H, *J =* 8.0 Hz), 7.20 (d, 2H, *J =* 8.8 Hz), 7.12 (s, 1H), 4.57 (m, 1H), 3.92 (m, 1H), 3.15 (s, 3H), 2.08 (m, 4H), 1.57 (m, 2H), 1.49 (m, 2H); ¹³C NMR (DMSO-*d*₆, 125 MHz)*δ* 161.3, 160.3, 151.4, 151.2, 134.3, 134.1, 133.8, 133.6, 132.3, 129.3, 127.7, 127.1, 124.9, 122.7, 121.4, 120.6, 120.1, 116.9, 115.8, 74.4, 47.7, 44.0, 29.2.

### (18) 4-(((1,4-trans)-4-((4-Oxo-7-(trifluoromethyl)-1,4-dihydroquinazolin-2-yl)amino)cyclohexyl)oxy)benzenesulfonamide (hereinafter referred to as Compound 18)

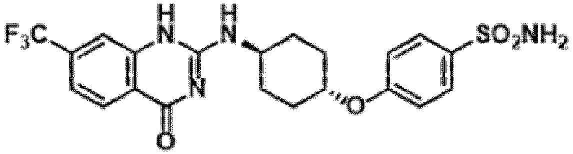

4-(((1,4-trans)-4-Aminocyclohexyl)oxy)benzenesulfonamide (104 mg, 0.39 mmol) and N,N-diisopropylethylamine (167 µL, 0.96 mmol) were added to DMF solution (3 mL) containing 2-chloro-7-(trifluoromethyl)quinazolin-4(1H)-one (80.0 mg, 0.32 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/n-hexane (3:2) and purified to obtain 22.2 mg (14%) of Compound 18 in white solid.

¹H NMR (DMSO-*d*₆, 500 MHz)*δ* 10.89 (s, 1H), 8.06 (d, 1H, *J =* 8.2 Hz), 7.73 (d, 2H, *J* = 8.9 Hz), 7.51 (s, 1H), 7.35 (d, 1H, *J* = 7.8 Hz), 7.19 (s, 2H), 7.11 (d, 2H, *J* = 8.9 Hz), 6.53 (s, 1H), 4.50 (m, 1H), 3.89 (m, 1H), 2.08 (m, 4H), 1.54 (m, 2H), 1.47 (m, 2H); ¹³C NMR (DMSO-*d*₆, 125 MHz)*δ* 161.3, 159.9, 151.4, 151.0, 136.0, 134.2, 134.0, 127.8, 127.7, 127.1, 124.9, 122.8, 121.4, 120.6, 120.1, 117.1, 115.4, 74.4, 48.0, 29.5, 29.4.

### (19) 2-(((1,4-trans)-4-(4-Chlorophenoxy)cyclohexyl)amino)-7-(trifluoromethyl)quinazolin-4(1H)-one (hereinafter referred to as Compound 19)

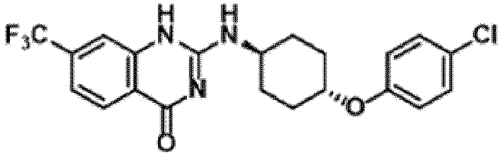

(1,4-trans)-4-(4-Chlorophenoxy)cyclohexan-1-amine (87.0 mg, 0.48 mmol) and N,N-diisopropylethylamine (167 µL, 0.96 mmol) were added to DMF solution (3 mL) containing 2-chloro-7-(trifluoromethyl)quinazolin-4(1H)-one (80.0 mg, 0.32 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/CH₂Cl₂ (1:4) and purified to obtain 16.7 mg (12%) of Compound 19 in white solid.

¹H NMR (DMSO-*d*₆, 500 MHz)*δ* 10.92 (s, 1H), 8.05 (d, 1H, *J =* 8.2 Hz), 7.51 (s, 1H), 7.35 (d, 1H, *J =* 8.1 Hz), 7.30 (d, 2H, *J =* 7.8 Hz), 6.99 (d, 2H, *J =* 8.0 Hz), 6.57 (s, 1H), 4.36 (m, 1H), 3.88 (m, 1H), 2.06 (m, 2H), 2.05 (m, 2H), 1.51 (m, 2H), 1.44 (m, 2H); ¹³C NMR (DMSO-*d*₆, 125 MHz)*δ* 161.4, 156.2, 151.3, 151.1, 134.4, 134.1, 133.8, 133.6, 129.3, 127.6, 127.1, 124.9, 124.1, 122.7, 121.4, 120.6, 120.1, 117.4, 117.0, 74.4, 48.00, 29.52, 29.38.

### (20) 2-(((1,4-trans)-4-(4-Chlorophenoxy)cyclohexyl)amino)-7-(trifluoromethyl)quinazolin-4(1H)-one (hereinafter referred to as Compound 20)

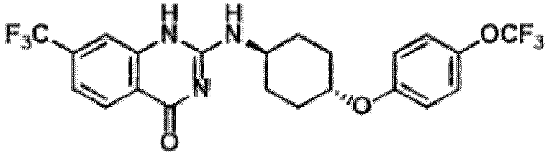

(1,4-trans)-4-(4-(Trifluoromethoxy)phenoxy)cyclohexan-1-amine (166 mg, 0.60 mmol) and N,N-diisopropylethylamine (210 µL, 1.21 mmol) were added to DMF solution (3 mL) containing 2-chloro-7-(trifluoromethyl)quinazolin-4(1H)-one (100 mg, 0.40 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/CH₂Cl₂ (1:3) and purified to obtain 6 mg (14%) of Compound 20 in white solid.

¹H NMR (DMSO-*d*₆, 500 MHz)*δ* 10.84 (s, 1H), 8.05 (d, 1H, *J =* 8.2 Hz), 7.51 (s, 1H), 7.35 (d, 1H, *J =* 8.1 Hz), 7.27 (d, 2H, *J =* 8.7 Hz), 7.06 (d, 2H, *J =* 9.1 Hz), 6.46 (s, 1H), 4.39 (m, 1H), 3.89 (m, 1H), 2.07 (m, 2H), 2.05 (m, 2H), 1.52 (m, 2H), 1.45 (m, 2H); ¹³C NMR (DMSO-*d*₆, 125 MHz)*δ* 161.1, 156.2, 151.3, 150.8, 141.6, 141.6, 134.4, 134.2, 133.9, 133.7, 127.6, 127.1, 124.9, 123.2, 122.7, 122.5, 121.4, 121.2, 120.5, 120.1, 119.2, 117.2, 117.0, 116.8, 74.6, 48.0, 29.5, 29.4.

### (21) 7-Nitro-2-(((1,4-trans)-4-(4-(trifluoromethyl)phenoxy)cyclohexyl)amino)quinazolin-4(1H)-one (hereinafter referred to as Compound 21)

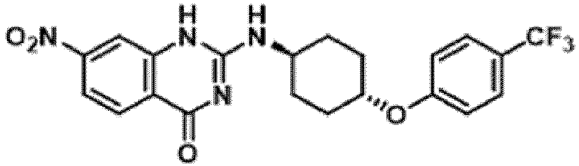

(1,4-trans)-4-(4-(Trifluoromethyl)phenoxy)cyclohexan-1-amine (259 mg, 1.00 mmol) and N,N-diisopropylethylamine (350 µL, 2.01 mmol) were added to DMF solution (5 mL) containing 2-chloro-7-nitroquinazolin-4(1H)-one (150 mg, 0.67 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 139 mg (47%) of Compound 21 in yellow solid.

¹H NMR (DMSO-*d*₆, 500 MHz)*δ* 10.96 (s, 1H), 8.09 (d, 1H, *J =* 8.7 Hz), 7.93 (s, 1H), 7.80 (d, 1H, *J =* 7.7 Hz), 7.63 (d, 2H, *J =* 8.0 Hz), 7.15 (d, 2H, *J =* 8.2 Hz), 6.60 (s, 1H), 4.52 (m, 1H), 3.91 (m, 1H), 2.09 (m, 1H), 1.57 (m, 2H), 1.49 (m, 2H); ¹³C NMR (DMSO-*d*₆, 125 MHz)*δ* 160.9, 160.3, 151.8, 151.4, 151.2, 127.8, 127.0, 127.0, 126.9, 125.7, 123.5, 121.7, 121.4, 121.2, 121.0, 120.7, 120.5, 119.2, 115.8, 114.9, 74.3, 48.0, 29.4, 29.3.

### (22) 7-Nitro-2-(((1,4-trans)-4-(4-nitrophenoxy)cyclohexyl)amino)quinazolin-4(1H)-one (hereinafter referred to as Compound 22)

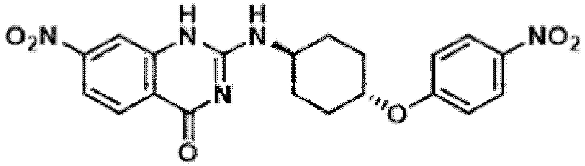

(1,4-trans)-4-(4-Nitrophenoxy)cyclohexan-1-amine (188 mg, 0.80 mmol) and N,N-diisopropylethylamine (277 µL, 1.59 mmol) were added to DMF solution (4 mL) containing 2-chloro-7-nitroquinazolin-4(1H)-one (120 mg, 0.53 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 76.0 mg (34%) of Compound 22 in yellow solid.

¹H NMR (DMSO-*d*₆, 500 MHz)*δ* 10.94 (s, 1H), 8.18 (d, 2H, *J =* 8.7 Hz), 8.07 (d, 1H, *J =* 8.5 Hz), 7.92 (s, 1H), 7.79 (d, 1H, *J =* 8.0 Hz), 7.18 (d, 2H, *J =* 8.7 Hz), 6.63 (s, 1H), 4.60 (m, 1H), 3.91 (m, 1H), 2.10 (m, 4H), 1.59 (m, 2H), 1.50 (m, 2H); ¹³C NMR (DMSO-*d*₆, 125 MHz)*δ* 163.0, 161.2, 151.9, 151.4, 140.6, 128.2, 126.1, 121.7, 119.2, 115.8, 115.5, 115.0, 75.2, 48.1, 29.4, 29.4.

### (23) 4-(((1,4-trans)-4-((7-Nitro-4-oxo-1,4-dihydroquinazolin-2-yl)amino)cyclohexyl)oxy)benzonitrile (hereinafter referred to as Compound 23)

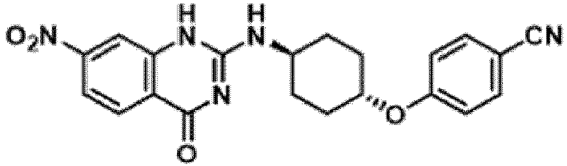

4-(((1,4-trans)-4-Aminocyclohexyl)oxy)benzonitrile (173 mg, 0.80 mmol) and N,N-diisopropylethylamine (277 µL, 1.59 mmol) were added to DMF solution (4 mL) containing 2-chloro-7-nitroquinazolin-4(1H)-one (120 mg, 0.53 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 80.4 mg (37%) of Compound 23 in yellow solid.

¹H NMR (DMSO-*d*₆, 500 MHz)*δ* 10.92 (s, 1H), 8.07 (d, 1H, *J =* 8.7 Hz), 7.91 (s, 1H), 7.78 (dd, 1H, *J =* 8.6, 1.6 Hz), 7.74 (d, 2H, J= 8.7 Hz), 7.14 (d, 2H, *J =* 8.8 Hz), 6.67 (s, 1H), 4.53 (m, 1H), 3.90 (m, 1H), 2.08 (m, 4H), 1.56 (m, 2H), 1.48 (m, 2H); ¹³C NMR (DMSO-*d*₆, 125 MHz)*δ* 161.0, 151.7, 151.3, 134.2, 128.1, 121.7, 119.2, 116.3, 114.8, 102.5, 74.5, 48.0, 29.4, 29.3.

### (24) 2-(((1,4-trans)-4-(4-(Methylsulfonyl)phenoxy)cyclohexyl)amino)-7-nitroquinazolin-4(1H)-one (hereinafter referred to as Compound 24)

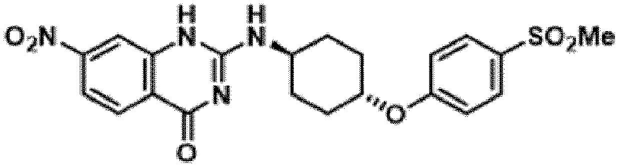

(1,4-trans)-4-(4-(Methylsulfonyl)phenoxy)cyclohexan-1-amine (173 mg, 0.80 mmol) and N,N-diisopropylethylamine (229 µL, 1.32 mmol) were added to DMF solution (3 mL) containing 2-chloro-7-nitroquinazolin-4(1H)-one (100 mg, 0.44 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 48.8 mg (24%) of Compound 24 in yellow solid.

¹H NMR (DMSO-*d*₆, 500 MHz)*δ* 8.07 (d, 1H, *J =* 8.6 Hz), 7.92 (s, 1H), 7.82 (d, 2H, *J =* 8.4 Hz), 7.79 (d, 1H, *J* = 8.8 Hz), 7.19 (d, 2H, *J* = 8.4 Hz), 6.78 (s, 1H), 4.54 (m, 1H), 3.92 (m, 1H), 3.15 (s, 3H), 2.09 (m, 4H), 1.57 (m, 2H), 1.50 (m, 2H); ¹³C NMR (DMSO-*d*₆, 125 MHz)*δ* 161.3, 161.1, 151.7, 151.3, 132.3, 130.1, 129.3, 128.1, 121.7, 119.1, 115.8, 114.9, 74.6, 48.0, 44.0, 29.4, 29.3.

### (25) 6-Chloro-2-(((1,4-trans)-4-(4-(trifluoromethyl)phenoxy)cyclohexyl)amino)quinazolin-4(1H)-one (hereinafter referred to as Compound 25)

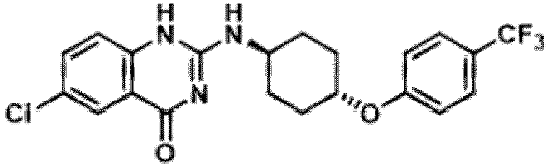

(1,4-trans)-4-(4-(Trifluoromethyl)phenoxy)cyclohexan-1-amine (331 mg, 1.28 mmol) and N,N-diisopropylethylamine (444 µL, 2.55 mmol) were added to DMF solution (7 mL) containing 2,6-dichloroquinazolin-4(1H)-one (184 mg, 0.85 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 45.0 mg (12%) of Compound 25 in light yellow solid.

¹H NMR (DMSO-*d*₆, 400 MHz)*δ* 11.00 (s, 1H), 7.79 (s, 1H), 7.61 (d, 2H, *J* = 7.0 Hz), 7.54 (d, 1H, *J =* 6.7 Hz), 7.26 (d, 1H, *J =* 8.1 Hz), 7.14 (d, 2H, *J =* 7.2 Hz), 6.74 (s, 1H), 4.51 (m, 1H), 3.87 (m, 1H), 2.06 (m, 4H), 1.50 (m, 4H); ¹³C NMR (DMSO-*d*₆, 100 MHz)*δ* 160.8, 160.3, 150.3, 150.0, 134.2, 128.7, 127.0, 127.0, 126.8, 126.0, 125.3, 124.8, 123.3, 121.3, 121.0, 120.7, 120.6, 120.4, 118.5, 115.8, 74.3, 47.7, 30.7, 29.3.

### (26) 6-Chloro-2-(((1,4-trans)-4-(4-nitrophenoxy)cyclohexyl)amino)quinazolin-4(1H)-one (hereinafter referred to as Compound 26)

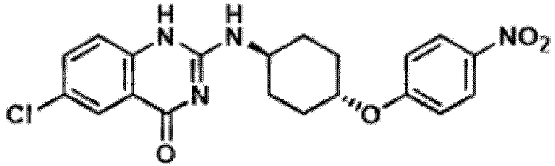

(1,4-trans)-4-(4-(Trifluoromethyl)phenoxy)cyclohexan-1-amine (156 mg, 0.66 mmol) and N,N-diisopropylethylamine (230 µL, 1.32 mmol) were added to DMF solution (3 mL) containing 2,6-dichloroquinazolin-4(1H)-one (100 mg, 0.44 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 52.7 mg (29%) of Compound 26 in light brown solid.

¹H NMR (DMSO-*d*₆, 500 MHz)*δ* 8.18 (d, 2H, *J =* 9.2 Hz), 7.80 (d, 1H, *J* = 2.2 Hz), 7.56 (dd, 1H, *J* = 8.7, 2.2 Hz), 7.27 (d, 1H, *J =* 8.7 Hz), 7.17 (d, 2H, *J =* 9.1 Hz), 6.42 (s, 1H), 4.58 (m, 1H), 3.87 (m, 1H), 2.08 (m, 4H), 1.57 (m, 2H), 1.47 (m, 2H); ¹³C NMR (DMSO-*d*₆, 125 MHz)*δ* 163.0, 161.1, 150.3, 150.0, 140.6, 134.3, 126.8, 126.0, 125.5, 124.9, 118.5, 115.8, 75.2, 47.8, 29.4.

### (27) 4-(((1,4-trans)-4-((6-Chloro-4-oxo-1,4-dihydroquinazolin-2-yl)amino)cyclohexyl)oxy)benzonitrile (hereinafter referred to as Compound 27)

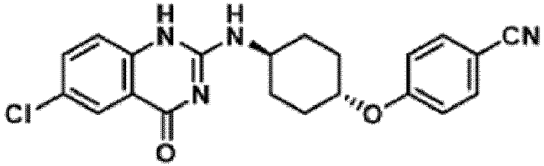

4-(((1,4-trans)-4-Aminocyclohexyl)oxy)benzonitrile (143 mg, 0.66 mmol) and N,N-diisopropylethylamine (230 µL, 1.32 mmol) were added to DMF solution (3 mL) containing 2,6-dichloroquinazolin-4(1H)-one (100 mg, 0.44 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 64.5 mg (37%) of Compound 27 in white solid.

¹H NMR (DMSO-*d*₆, 500 MHz)*δ* 10.78 (s, 1H), 7.80 (d, 1H, *J =* 2.5 Hz), 7.74 (d, 2H, *J =* 8.9 Hz), 7.55 (dd, 1H, *J =* 8.8, 2.6 Hz), 7.27 (d, 1H, *J =* 8.8 Hz), 7.13 (d, 2H, *J =* 9.0 Hz), 6.39 (s, 1H), 4.52 (m, 1H), 3.86 (m, 1H), 2.06 (m, 4H), 1.54 (m, 2H), 1.45 (m, 2H); ¹³C NMR (DMSO-*d*₆, 125 MHz)*δ* 161.1, 161.0, 150.4, 150.0, 134.3, 134.2, 126.8, 125.4, 124.8, 119.2, 118.4, 116.4, 102.5, 74.6, 47.8, 29.4.

### (28) 6-Chloro-2-(((1,4-trans)-4-(4-(methylsulfonyl)phenoxy)cyclohexyl)amino)quinazolin-4(1H)-one (hereinafter referred to as Compound 28)

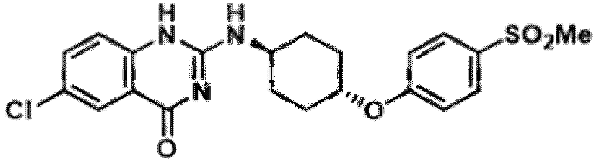

(1,4-trans)-4-(4-(Methylsulfonyl)phenoxy)cyclohexan-1-amine (133 mg, 0.50 mmol) and N,N-diisopropylethylamine (172 µL, 0.99 mmol) were added to DMF solution (2 mL) containing 2,6-dichloroquinazolin-4(1H)-one (70.0 mg, 0.33 mmol) at ambient temperature. After stirring at 80°C for 17 hours, the reaction mixture was quenched with water (H₂O) and extracted with ethyl acetate (EtOAc). The combined organic layers were dried over MgSO₄ and concentrated in vacuo. The residue was recrystallized from EtOAc/MeOH (10:1) and purified to obtain 70.8 mg (48%) of Compound 28 in ivory solid.

¹H NMR (DMSO-*d*₆, 500 MHz)*δ* 10.82 (s, 1H), 7.82 (d, 2H, *J =* 8.8 Hz), 7.80 (m, 1H), 7.56 (d, 1H, *J =* 8.6 Hz), 7.27 (d, 1H, *J =* 8.8 Hz), 7.19 (d, 2H, *J =* 8.6 Hz), 6.45 (s, 1H), 4.55 (m, 1H), 3.87 (m, 1H), 3.15 (s, 3H), 2.07 (m, 4H), 1.56 (m, 2H), 1.46 (m, 2H); ¹³C NMR (DMSO-*d*₆, 125 MHz)*δ* 161.4, 161.1, 150.3, 150.0, 134.3, 132.3, 129.3, 126.8, 125.5, 124.9, 118.5, 115.8, 74.6, 47.8, 44.0, 29.4.

### [Example 1] Analysis of fold changes in eIF2α phosphorylation for 2-((trans-4-(4-aryloxy)cyclohexyl)amino)quinazolinone derivatives

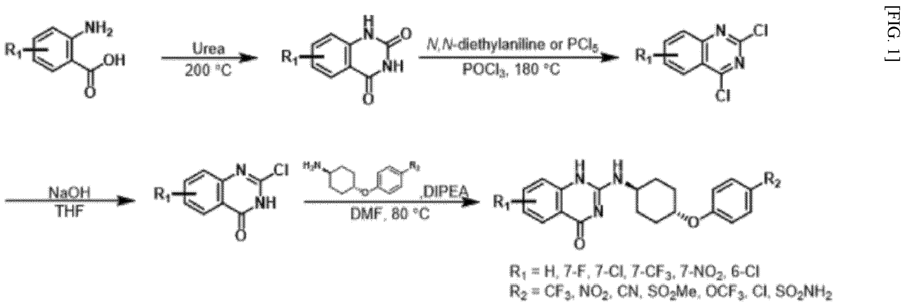

PathScan phospho-eIF2α (Ser51) Sandwich ELISA Kit #7286 from Cell Signaling was used. K562 cell lines were cultured in serum-free RPMI medium at 37°C in the presence of 5% CO₂. Compounds 1 to 28 prepared in the above Synthesis Examples were treated to cells at a concentration of 10 µM. 6 hours after compound treatment, cells were lysed with the lysis buffer included in the kit, and 50 µg of protein was quantified and obtained. After that, the absorbance was obtained by performing the ELISA assay according to the manufacturer's protocol. The absorbance for each compound obtained was normalized to the value of control (DMSO-treated) set to 1. The results are as shown in FIG. 1.

### [Example 2] Analysis of the anti-proliferative activity of 2-((trans-4-(4-aryloxy)cyclohexyl)amino)quinazolinone derivatives for K562

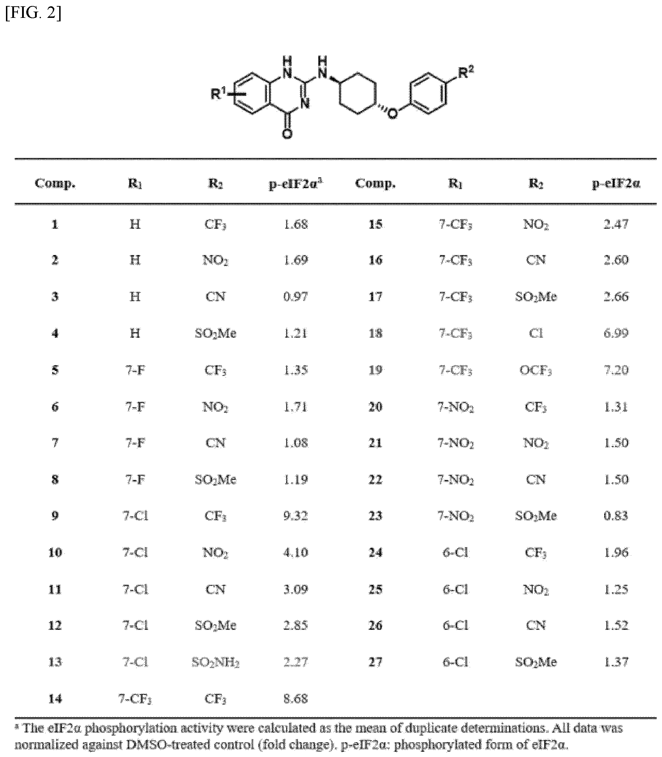

CCK-8 assay (cell counting kit-8 assay, Dojindo, Kumamoto, Japan) was performed. K562 cell lines were cultured in RPMI medium supplemented with 10% FBS and 1% ABAM at 37°C in the presence of 5% CO₂. The cells were treated with the Compounds 1, 9 to 12, 14 to 17, and 25 at concentrations of 0 µM, 1.25 µM, 2.5 µM, 5 µM, 10 µM, and 20 µM, respectively. After undergoing culture for 3 days, 10 µL of CCK-8 solution was treated, followed by culture for 4 hours. Afterwards, the IC₅₀ value of each compound was obtained by measuring the absorbance at 450 nm. The results are as shown in FIG. 2.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A compound selected from a 4-(((1,4-trans)-4-((4-oxo-7-(trifluoromethyl)-1,4-dihydroquinazolin-2-yl)amino)cyclohexyl)oxy)benzenesulfonamide compound, a pharmaceutically acceptable salt thereof, a solvate thereof, or a stereoisomer thereof.

2. A pharmaceutical composition for preventing or treating a cancer, comprising the compound according to claim 1 as an active ingredient.

3. The pharmaceutical composition of claim 2, wherein the compound promotes phosphorylation of eukaryotic translation initiation factor 2α (eIF2α).

4. The pharmaceutical composition of claim 2, wherein the cancer is one or more selected from the group consisting of blood cancer, breast cancer, sarcoma, or brain cancer.

5. A pharmaceutical composition for a metabolic anticancer agent, comprising the compound according to claim 1 as an active ingredient.

6. The pharmaceutical composition of claim 5, wherein the compound blocks metabolism of cancer cells by promoting phosphorylation of eukaryotic translation initiation factor 2α (eIF2α).

7. A method of inhibiting cancer cell metabolism, comprising administering the compound according to claim 1.

8. The method of claim 7, wherein the compound blocks cancer cell metabolism by promoting phosphorylation of eukaryotic translation initiation factor 2α (eIF2α).

9. A health functional food composition for preventing or ameliorating a cancer, comprising the compound according to claim 1 as an active ingredient.
